# EUROPEAN PATENT SPECIFICATION

(11) **EP 2 952 885 B1**
(45) Date of publication and mention of the grant of the patent: **27.07.2016**
(21) Application number: 14001902.7
(22) Date of filing: 02.06.2014
(51) Int. Cl.: G01N 33/00, G01N 27/12

(54) **Gas sensor**
Gassensor
Capteur de gaz

(43) Date of publication of application: 09.12.2015
(73) Proprietor: Sensirion AG, 8712 Stäfa (CH)
(72) Inventor: Hitzbleck, Martina, 8712 Stäfa (CH); Mücke, Ulrich, 8712 Stäfa (CH); Mayer, Felix, 8712 Stäfa (CH); Bürgi, Lukas, 8712 Stäfa (CH); Graf, Markus, 8712 Stäfa (CH)
(74) Representative: Toleti, Martin

(56) References cited:
- EP-A1- 2 713 157
- WO-A2-99/53287
- WO-A2-2012/003348
- WO-A2-2013/095730
- WO-A2-2014/007603
- DE-A1- 19 642 453
- DE-A1-102006 019 534
- US-A- 5 510 013

## Description

### Technical Field

The invention relates to a gas sensor and to a method for manufacturing a gas sensor.

### Background Art

A gas sensor performs a detection of chemical substances or compounds which are also denoted as analytes contained in a gas, or possibly in a fluid, supplied to the gas sensor. In case multiple different analytes shall be detected by a gas sensor, the gas sensor may comprise multiple sensor cells with each sensor cell being designed for detecting one or more of the subject analyses.

WO 99/53287 A2 addresses techniques used to fabricate and manufacture sensors to detect analytes. An analyte is sensed by sensors that output electrical signals in response to the analyte. The electrical signals are preprocessed by filtering and amplification. In an embodiment, this preprocessing includes adapting the sensor and electronics to the environment in which the analyte exists. The electrical signals are further processed to classify and identify the analyte, which may be by a neural network.

EP 2 713 157 A1 refers to a method for manufacturing a chemical sensor with multiple sensor cells on a substrate. An expansion inhibitor is applied to the substrate for preventing a sensitive material to be applied to an area on the substrate for building a sensitive film of a sensor cell to expand from said area. The sensitive material is provided and the sensitive film is built by contactless dispensing the sensitive material to said area.

DE 10 2006 019534 A1 relates to a capacitively or conductively acting micro-sensor having a first and a second electrode, at least one sensitive layer or at least one functionalized surface between the electrodes, and an insulating substrate on which the electrodes and the at least one sensitive layer or the at least one functionalized surface are arranged. The electrodes are located in approximately the same distance from the substrate. Electrode surfaces adjacent to the sensitive layer are provided with an inclination relative to an axis perpendicular to the substrate surface, wherein the inclination of the electrode is oriented such that the distance between the respective opposite electrode surfaces decreases towards the substrate.

WO 2013/095730 A2 discloses a muitimode gas sensor platform with an array of electrode pairs oriented on a substrate and a plurality of detection zones, wherein at least a portion of individual electrode pairs are separately addressable. Each detection zone can comprise at least one set of individual electrode pairs within the array, where the individual electrode pairs have organic nanofibers uniformly deposited thereon. The organic nanofibers can be responsive to association with a corresponding target material and at least one detection zone can be electronically responsive to the corresponding target material.

### Disclosure of the Invention

It is desired to provide a way for manufacturing a gas sensor that results in a high quality and reliable gas sensor as well as such a high quality and reliable gas sensor.

The problem is solved by a gas sensor with a set of one or more sensor cells. A sensor cell may be understood as an entity of the gas sensor which may be read individually. Each sensor cell of the set comprises a sensitive film built from a sensitive material covering an area of the substrate which area does not extend beyond a maximal admissible coverage area for the sensor cell that may be defined upfront. When bringing the sensitive material onto the substrate, it is desired that the sensitive material does not extend outside such maximal admissible coverage area designated for the sensitive film. For this purpose, it is envisaged to provide one or more elevated structures in said maximal admissible coverage area for preventing the sensitive material to expand therefrom when being applied thereto. It is noted that the one or more elevated structures are arranged in maximal admissible coverage area, i.e. their footprint is within maximal admissible coverage area. For this reason a top surface of the one or more elevated structures its likely to be covered by the sensitive material. An elevated structure is perceived as an elevation if the sensitive material is deposited to a level of the substrate from which the expansion inhibitors protrude. In another example, which is not claimed, the elevated structure may rather be perceived as indentation in case the sensitive material is deposited to a level of the substrate that coincides with the top level of the elevations.

It is intended that an expansion of the sensitive material at least stops at the outmost elevated structure/s, and specifically at a falling edge thereof owed to surface tension effects in the sensitive material when covering the top surface thereof. Specifically, such outmost elevated structure comprises a first side face facing the sensitive material when expanding. A second side face is averted from the first side face and therefore does not face the expanding sensitive material. The first side face may finally be covered by the sensitive material as may be the top surface of this outmost elevated structure. However, owed to surface tension the second side face may not be covered and the expansion of the sensitive material can be stopped at the falling edge.

The, or a number of, or all of the elevated structures are made from an insulating coating of the substrate, and specifically are made from one of silicon nitride and silicon oxide. Given that the substrate is defined as also encompassing layers of material, - such as CMOS layers, if any - deposited onto a bulk substrate material, the at least one elevated structure can be manufactured by standard CMOS processes such as lithography and etching. In a preferred embodiment, a top insulating layer of the substrate, such as a passivation layer in form of a silicon nitride layer may be used for manufacturing the at least one elevated structure. A recess may be etched into the insulating layer. Any elevated structures to be generated are exempted from etching and remain as structures protruding from the recess. Preferably, the recess is dimensioned to exceed the maximal admissible coverage area the sensitive film shall finally cover at maximum such that after having fabricated the sensitive film, at least a trench remains between the sensitive film and a rim of the insulating layer defining the recess.

The area covered by the sensitive film may in some examples be less than the maximal admissible coverage area in case any of the inner elevated structures may already cause the expansion of the sensitive material to stop. In such case, other elevated structures may remain uncovered by the sensitive material and may remain standsing free between the sensitive film and the rim.

In a different embodiment, an electrically conducting layer of the substrate may be used for manufacturing one or more elevated structures from. Such layer may be a metal layer, for example made from one of gold, platinum and aluminum. This elevated structure may at the same time serve as electrode for electrically contacting the sensitive film. Hence, in this embodiment, the elevated structure/s are connected to an electrical contact where a voltage or a current can be applied to for reading the sensor cell. In a different embodiment, the conducting property of the elevated structure is not utilized, and the elevated structure/s are disconnected from any voltage or current source. The structures may remain at floating potential and do not serve for any other purpose than inhibiting expansion of the sensitive material. In a different embodiment, an elevated structure may only be covered by an electrically conducting material while a core of the elevated structure is of non-conducting property.

In a different embodiments, elevated structures of different material may be mixed within the maximal admissible coverage area assigned to a sensor cell. For example, an inner portion thereof comprises one or more elevated structures made from or covered by an electrically conducting material, while an outer portion of the area surrounding the inner portion comprises one or more elevated structures made from an insulating coating.

In another embodiment, a portion of the one or more elevated structures is arranged outside a region that is covered by an electrode pattern for measuring a property of the sensitive film. Hence, the area of the sensitive film does not necessarily match with the region the electrodes cover. For example, the electrode pattern may be arranged within a square while the maximal admissible coverage area is defined as a circle, e.g. extending outside the square region defined by the electrodes As a result, a portion or a number of one or more elevated structures is arranged outside this region. The electrode pattern may reside underneath the elevated structure/s, or may at the same time serve as elevated struc-. tures as described above.

In a preferred embodiment, each sensor cell of the set comprises more than two elevated structures, and preferably three, four, or five, or any other number between three and ten. An elevated structure in this context is considered as an elevation not linked to another elevation. Each elevated structure may preferably take the form of a closed ring, such as the circumference of a circle or the circumference of a rectangle, with the ring structures having different radii or diameters and being arranged within each other. Provided the sensitive material is introduced to a centre of the maximal admissible coverage area, the material tends to expand radially from this centre. When having arranged such repeating ring structures, the sensitive material first has to overcome the first ring, then the second ring, and so on, until it at least stops at the outmost ring owed to the surface tension effect. However, the material may stop at any inner ring, be it its rising or its falling edge, and then only cover an area that is smaller than the maximal admissible coverage area.

In another embodiment, a set of elevated structures is provided with more than ten, and especially more than fifty elevated structures. Preferably, a footprint of each elevated structure of the set is less than 35 µm x 35 µm, and in particular is less than 5 µm x 5 µm, and in particular is less than 1 µm x 1 µm. A height of such elevated structure may be equal to or more than 0.5 µm, and in particular may be between 0.5 and 5 µm, and in particular is less than 35 µm. The elevated structures of the set may take the shape of an uprising member with a footprint significantly smaller - e.g. more than a hundred times smaller - than the area of the sensitive film. Those uprising members may take different 3D shapes subject to the geometry of their footprint. The footprint of an individual elevated structure may be of one of: an oval, a circle, a rectangle, a square, a triangle. The 3D shape of a corresponding elevated structures may be one of a pillar, a post, a prism. These elevated structures may be manufactured from SiN, for example, or from a photoresist, such as SU8, or from a dry film resist, such as TMMF. Any of the elevated structures addressed may be manufactured from one of these materials.

In a preferred embodiment, the elevated structures comprise a set of elevated structures such as described in the previous paragraph, and in addiction one or more elevated ring structures, e.g. encircling the set of elevated structures.

It was noticed that the more elevated structures are arranged in a direction of the expansion of the sensitive material, the more energy is required for the sensitive material to expand. This is owed to an increase of a surface to be wet by the material invoked by the number of structures. As a result, in such embodiments higher contact angles can be achieved wherein a contact angle is defined as angle at which the sensitive film meets a top surface of an elevated structure. A high contact angle is desired, for localizing sufficiently large volumes on the sensor, for example ∼100 pL in an area of ~70 um diameter.

In a preferred embodiment of the gas sensor, the area covered by the sensitive film is dimensioned at less than 100 µm times 100 µm. It is preferred that this area is a circle, especially when its ohmic resistance is measured for detecting analytes. Preferably, the sensitive film is arranged on a hotplate of the substrate, i.e. a region in which the substrate is thinned compared to other regions of the substrate. In a preferred embodiment, the sensitive material includes nano-particles in a suspension or solution, for example metal oxide nanoparticles, which is dispensed to the substrate and may also be referred to as ink. The solution or suspension may evaporate after deposition and the remainder of the sensitive material contributes to the sensitive film. Preferably, the gas sensor is a chemoresistive sensor containing a metal-oxide semiconductor material applied to a substrate and building a sensitive film therefrom. In another preferred embodiment, the sensitive material includes polymer material, especially a soluble polymer material, such as polyimide, polythiophene, polyurethane, or polyaniline.

According to another aspect of the invention, a method is provided for manufacturing a gas sensor with at least one sensor cell. A substrate is provided including a maximal admissible coverage area to build the sensor cell on. At least one elevated structure is manufactured in said maximal admissible coverage area for preventing a sensitive material to expand therefrom when being applied thereto. A sensitive film of the sensor cell is then built by contactless dispensing the sensitive material to said maximal admissible coverage area. During and after dispensing, the sensitive material extends and is prevented from expanding from said maximal admissible coverage area by the at least one elevated structure.

Other advantageous embodiments are listed in the dependent claims as well as in the description below. The described embodiments similarly pertain to the sensor and the method. Synergetic effects may arise from different combinations of the embodiments although they might not be described in detail.

### Brief Description of the Drawings

The embodiments defined above and further aspects, features and advantages of the present invention can also be derived from the examples of embodiments to be described hereinafter, and are explained with reference to the drawings. In the drawings it is illustrated in:
Figure 1 a sectional view of a gas sensor according to an embodiment of the present invention, in different manufacturing steps,
Figure 2 a sectional view of a gas sensor according to another embodiment of the present invention, in different manufacturing steps,
Figure 3 a top view on elevated structures of a gas sensor according to an embodiment of the present invention,
Figure 4 and Figure 5 each a schematic top view on elevated structures of a gas sensor according to an embodiment of the present invention,
Figure 6 a perspective view on elevated structures of a gas sensor according to an embodiment of the present invention,
Figure 7 a perspective view of an elevated structure as used in a gas sensor according to an embodiment of the present invention,
Figure 8 perspective views of different elevated structures as used in a gas sensor according to an embodiment of the present invention,
Figure 9 a top view on a gas sensor according to another embodiment of the present invention, and
Figure 10 a side cut of a gas sensor according to an embodiment of the present invention, and
Figure 11 a sectional view of a gas sensor according to another embodiment of the present invention.

### Modes for Carrying Out the Invention

A gas sensor may in one embodiment of the present invention comprise at least one sensitive film made from material being sensitive to one or more analytes. The number of sensitive films arranged separate from each other on a common substrate may define the number of sensor cells of a gas sensor. The gas sensor preferably is embodied as a sensor array comprising a set of two or more sensor cells, e.g. four sensor cells, wherein each sensor cell may include a sensitive film. A sensor cell may be understood as an entity of the gas sensor which may be read individually. Preferably, in the embodiment of the sensor array, each or at least some of the sensitive films are sensitive to different analytes. These sensitive films may accordingly be built from sensitive material of different composition. The corresponding sensitive films may exhibit different sensitivities from cell to cell such that each cell of the sensor array may be mainly sensitive to a specific analyte and as such may allow detecting the presence or absence or concentration of such analyte. "Mainly" in this context shall mean, that a sensor cell is more sensitive to the subject analyte than to other analytes. Preferably, in such array of sensor cells the sensitive films do not touch each other. The gas sensor performs a detection of chemical substances or compounds - collectively denoted as analytes - contained in a gas, or possibly in a fluid supplied to the gas sensor. Such analytes may include one or more of, for example, CO2, NOX, ethanol, CO, ozone, ammonia, formaldehyde, or xylene without limitation.

The gas sensor may comprise a sensitive material, e.g. in form of a film, the analyte may interact with. As a result, an electrical property of the sensor material may be modified upon interaction such as its electrical conductance, which principle preferably is applied in metal oxide gas sensors, for example. Then, the electrical property of a combination of the analyte and the sensor material is measured and allows a conclusion as to the analyte, such as by way of comparison to a property of the sensor material measured without the presence of the analyte. Specifically, the sensitive film/s may contain a metal oxide material, and in particular a semiconducting metal oxide material, and specifically may contain metal oxide materials of different composition per sensitive film. Such metal oxide material generally may include one or more of tin oxide, zinc oxide, titanium oxide, tungsten oxide, indium oxide and gallium oxide. Such metal oxides may be used for the detection of analytes such as VOCs, carbon monoxide, nitrogen dioxide, methane, ammonia or hydrogen sulphide. Metal oxide sensors are based on the concept that gaseous analytes interact with the metal oxide layer at elevated temperatures of the sensitive layer in the range of more than 100° Celsius, and specifically between 250°C and 350°Celsius. As a result of the catalytic reaction, the conductivity of the sensitive film may change which change can be measured. Hence, such gas sensors are also denoted as high temperature chemoresistors for the reason that a chemical property of the analyte is converted into an electrical resistance at high temperatures of the sensitive film. In a metal oxide gas sensor with multiple sensor cells, all sensitive films may be heated by a common heater, or each sensitive film may be heated by an individual heater.

Preferably, by means of such gas sensor a gas may be investigated at least as to the absence or presence of the subject analytes the gas sensor is sensitive to. Hence, the gas supplied to the gas sensor may be analyzed by means of the gas sensor as to if and which of the chemical substances or compounds the gas sensor is sensitive to are present in the gas supplied. A combination of analytes detected in the gas supplied may suggest for a certain odour or for a certain gas. It is always subject to a design of the gas sensor as to how many different analytes and/or how many different properties of an analyte the gas sensor is sensitive to. It is noted that for the different analytes the gas sensor is sensitive to it is not required to always measure the same property per analyte. Different properties may be measured for different analytes.

In the context of the present invention, a substrate shall include any platform for dispensing a suspension or solution of sensitive material to. The substrate may be one of a semiconductor, a glass or a ceramic substrate, or a polymer substrate, in particular a flexible polymer substrate, for example. However, the substrate may also include one or more layers deposited on a semiconductor substrate, for example, CMOS layers, on which layers the sensitive film finally is arranged. A certain area of the substrate that is also referred to as maximal admissible coverage area is designated to accept the sensitive material for finally forming a sensitive film. In view of the miniaturization of sensor chips, it is desired to minimize the area on/of the substrate covered by the sensitive material. In case of multiple sensor cells the sensitive films of these multiple sensor cells are desired to be arranged close to each other in view of space salving considerations. The area that shall be maximal allowed for building the sensitive film on may be defined upfront. It is preferred that the/these maximal admissible coverage areas are confined in order to save space on the substrate. Hence, it is preferred to provide an expansion inhibitor in form of at least one elevated structure protruding within said maximal admissible coverage area which elevated structure prevents sensitive material to be dispensed to escape from this maximal admissible coverage area, or in other words, to expand or to spill over from this maximal admissible coverage area.

When the sensitive material is dispensed to the assigned area, it is preferably dispensed in a contactless way. This means that there is no contact between a dispenser of the sensitive material and the substrate. Hence, a gap between the dispenser and the substrate has to be overcome by the sensitive material. In contrast, contact printing would be understood as pressing the dispenser against the substrate, such that for example a stamp covered by ink acting as dispenser is brought intro contact with the substrate for transferring the ink to the substrate. There is no gap to be overcome by the ink in contact printing. For implementing a contactless dispensing, it is preferred to provide the sensitive material as a liquid, e.g. in a container, and dispense the sensitive material in liquid form to the substrate on the designated areas. Contactless dispensing may preferably include jet dispensing wherein a continuous jet of liquid or discrete jet of liquid - e.g. in form of individual drops - is applied under pressure, e.g. by using a tight nozzle, to the substrate. Hence, jet printing may include an acceleration of the liquid in the dispenser to form a jet.

Hence, the dispensing approach may result in the sensitive material meet the substrate at high impact. This may be one of the reasons, why it is desired to provide an expansion inhibitor for confining the impacting jet to the maximal admissible coverage area. However, even after having dispensed the sensitive material to the substrate the sensitive material may at least for a limited period in time be flowable on the substrate and may want to escape the maximal admissible coverage area in order to reach its state of lowest energy. This is another instance when the expansion inhibitor prevents from such undesired escape of the sensitive material. In a beneficial secondary effect, even after a solvent may be evaporated from the sensitive film, e.g. by heating the substrate, and after the sensitive material is dried, e.g. by sintering, tempering, etc., and becomes a more or less solid sensitive film, the expansion inhibitor may support fixing the sensitive film in its position.

In the context of printing the sensitive material, the sensitive material may also be denoted as ink. An ink reservoir of a print head may be filled with the suspension, and the suspension may be jet printed onto the area on the substrate for depositing the sensitive material there. Specifically, the underlying ink jet printing technology may be one of the following:
- continuous ink jet printing;
- thermal ink jet printing;
- piezo ink jet printing.

However, in other embodiments, the contactless dispensing of sensitive material shall include one of screen printing, spraying, and jet dispensing. In one embodiment of contactless dispensing, a predefined amount of ink containing the sensitive material is applied as a single portion to the designated area for building one sensor cell. In a different embodiment, the predefined amount of ink may be split into multiple portions, e.g. between 2 and 100 portions, and may be dispensed to the designated area portion-wise for building one sensor cell. A portion may contain as less as a picolitre amount of ink.

It is preferred that all sensor cells of the set are monolithically integrated into a common sensors chip with a common substrate for all the sensor cells. Such monolithic sensor chip may be encapsulated and be arranged on and electrically connected to a conductor board. Such gas sensor chip may, due to its small size, be used in any portable electronic device such as a mobile phone, and in particular a smart phone, a handheld computer, an electronic reader, a tablet computer, a game controller, a pointing device, a photo or a video camera, or a computer peripheral, which listing is not limited, and may support the chemical and/or odour and/or gas identification as to its environment.

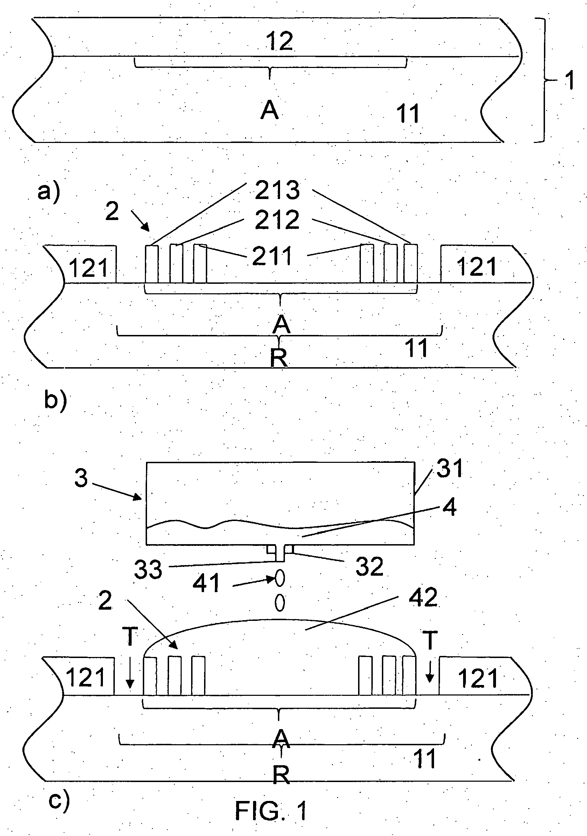

Figure 1 illustrates a sectional view of a gas sensor according to an embodiment of the present invention. According to diagram 1a), a substrate 1 is provided such as a semiconductor substrate comprising a bulk silicon 11 and an insulating coating 12 son top of the bulk silicon 11. However, there may be various other layers arranged in between the insulating coating 12 and the bulk silicon 11 which are not shown in the Figures. Specifically, all these layers including the insulating coating 12 may belong to a CMOS layer stack for integrating an electronic circuit. In a preferred embodiment, the insulating coating 12 is a SiN layer. An area A is designated as maximal admissible coverage area with respect to the substrate 1 for building a sensor cell on.

According to diagram 1b), the insulating coating 12 is structured and a recess R is etched having portions 121 of the insulating coating 12 remain as rim defining the recess R. In this embodiment, the elevated structures comprise three elevated rings 211, 212 and 213 arranged within each other.

In the next step shown in diagram 1c), a sensitive film 42 is built from a sensitive material 4 supplied by a print head 3 comprising a container 31 holding the liquid sensitive material 4. The print head 3 comprises a piezo actuator 32 arranged at a nozzle 33 of the print head 3 for forming and ejecting droplets 41 of the sensitive material 4 towards the substrate 1.

First a volume within the first ring 211 may be filled. Provided that sufficient sensitive material 4 is supplied by the print head 3, the first ring 211 may overflow and the sensitive material 4 pours into a gap between the first and the second ring 211, 212. Provided that the sensitive material is continued to be supplied from the print head 3, this gap is filled and the second ring 211 may overflow with the sensitive material 4 pouring into a gap between the second and the third ring 212, 213. The sensitive material 4 fills this gap and will spill over the third ring 213 and cover its top surface but does not pour into trench T owed to surface tension. In diagram 1c), it is suggested that multiple droplets 41 of the sensitive material 4 are supplied in sequence for building the sensitive film 42. In essence, the overall volume of sensitive material 4 is dimensioned such that its volume exceeds the volume within the first ring 42 in order to build a sensitive film 42 stopping at the falling edge of the outmost ring 213. In an alternate embodiment, a single droplet is supplied by the print head 3 in order to build the sensitive film 42 as shown. Here, the overall amount of ink is not quantized into smaller portions sequentially delivered by the print head 3, but is supplied at once. Provided that such droplet is directed towards the centre of the maximal admissible coverage area A or close by, the effect is the same in that the first ring 211 is flooded, etc., until surface tension will make the sensitive material 4 stop at the falling edge of the third ring 213.

The result of this process is a sensor cell SC as shown in diagram 1c) covering an area that coincides with the maximal admissible coverage area A. The sensor cell is separated from the rim 121 of the insulting coating 12 by trench T. Hence, the area covered by the sensor cell SC is smaller than the recess R etched into the insulating coating 12. However, in another embodiment, the sensitive material may even not make it to the third ring 213 and, e.g. stop at the falling edge of the second ring 212. In this embodiment, the area covered by the sensitive film 42 finally is smaller than the maximal admissible coverage area A.

The above teaching can, of course, be applied to different geometries of elevated structures 2, and to a different number of elevated structures 2.

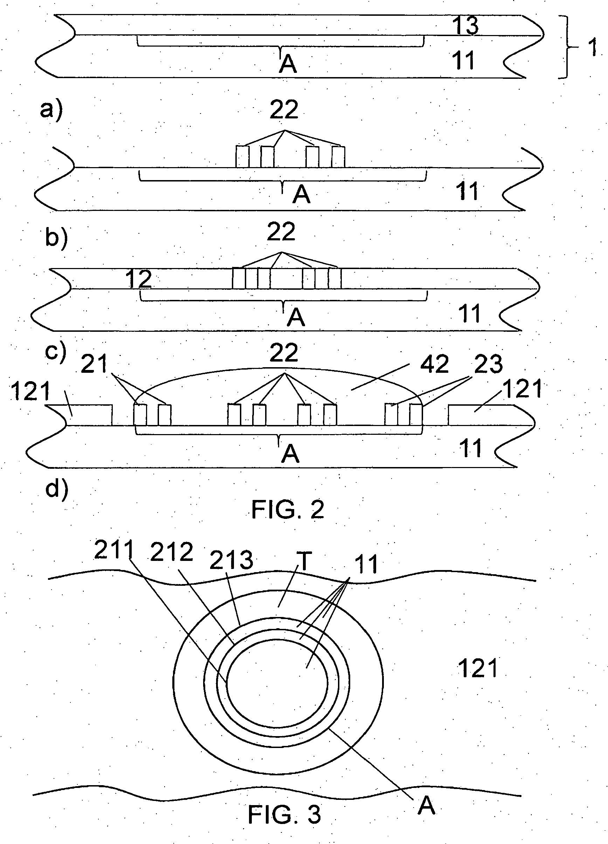

Figure 2 illustrates a sectional view of a gas sensor according to a different embodiment of the present invention. According to diagram 2a), a substrate 1 is provided such as a semiconductor substrate comprising a bulk silicon 11. A metal layer 13 is applied to the bulk silicon 11. Again, various other layers may be arranged in between the metal layer 13 and the bulk silicon 11. Again, all these layers including the metal layer 13 may belong to a CMOS layer stack for integrating an electronic circuit therein. In a preferred embodiment, the metal layer 13 may be made from aluminium. The maximal admissible coverage area A is designated with respect to the substrate 1 for building a sensor cell on.

In the following, the metal layer 13 is structured for building electrodes 22, such as interdigital electrodes, see diagram 2b.). In a next step, an insulating coating 12 is applied to the substrate 1, see diagram 2c). Additional elevated structures 21 are generated by etching the insulating coating 12. The additional elevated structures 21 may again be rings or have a different shape. As can be derived from diagram 2d), the elevated structures 22 and 21 protrude from the substrate 1 in form of electrode fingers made from metal and in form of two rings made from the insulating material. The elevated structures 21 and 22 in combination serve as expansion inhibitors for the sensitive material 4 to be applied. The resulting sensor film 42 is shown in diagram 2d). The sensitive material 4 is prevented from pouring into trench T by surface tension in combination with a granular dosage of the sensitive material 4.

Figure 3 shows a top view on elevated structures of a gas sensor according to an embodiments the present invention, and preferably according to the embodiment shown in Figure 1. The sensitive material is not applied yet to the substrate. Hence the top view may correspond to diagram 1b). In this top view, the rim 121 of the insulating coating is separated from the outmost ring 213 by trench T. Each ring 211, 212, 213 is referred to as a line only, such that between two lines representing rings the bulk silicon 11 or any other material under-neath the insulating coating may be seen in top view.

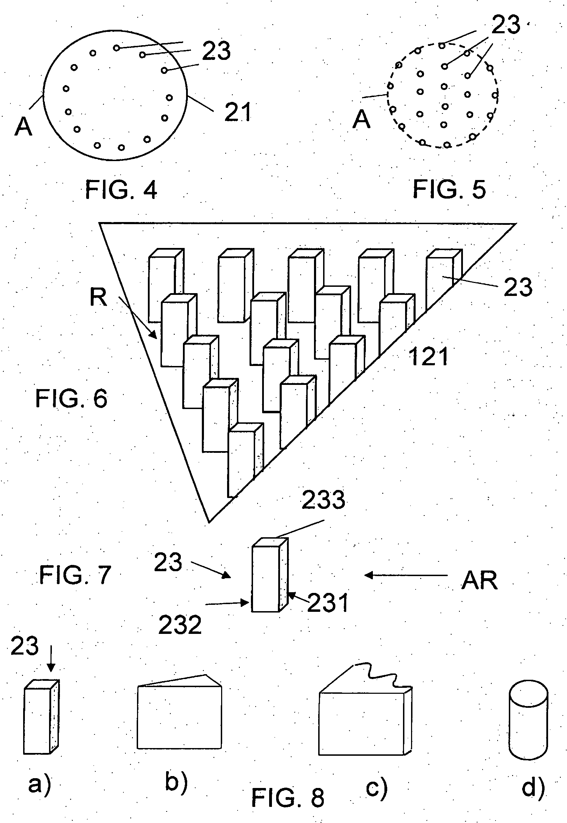

Figure 4 shows a schematic top view on elevated structures of a gas sensor according to an embodiment of the present invention. The elevated structures comprise a ring 21, and a set of multiple individual small-size elevated structures 23 denoted by the small circles. The set of small size elevated structures may be regarded as protruding members. Each elevated structure of the set supports limiting an expansion of the sensitive material given that surface tensions are invoked.

In contrast to the embodiment of Figure 4, the embodiment of Figure 5 no longer comprises an elevated ring, but solely provides the set of multiple small-size elevated structures which in combination act as expansion inhibitor. The maximal admissible coverage area A for the sensor cell is defined by the outer ring of elevated structures of the set.

Figure 6 shows a perspective view on a set of elevated structures in form of posts 23. The posts 23 are arranged within a recess R of an insulating coating. Hence, the posts 23 are made from the insulating coating material as well. The recess R is of triangle shape but may be of any other geometry. The ink may be supplied to the centre of the triangle. Again the ink shall stop at least at outer posts 23 thereby leaving a trench uncovered by sensitive material between the outmost row of posts 23 and the rim 121.

From the Figures 1 and 6 it becomes apparent, that the elevated structures for multiple sensor cells on the same substrate can be manufactured in a single structuring and etching step without the need to apply any additive processes. The sensitive films of various sensor cells may be arranged in recesses in the insulating layer next to each other. The various sensor cells are separated from each other by the rims defining the recesses.

Figure 7 shows a perspective view of an individual elevated structure such as a post 23 as may be used in the embodiment of Figure 6. The post 23 has a first side face 213 facing the ink when being exposed thereto under the assumption that the ink is applied to a location right to the first side face 231 and expanding in direction of arrow AR. A second side face 232 of the post 23 is averted from the first side face 231. A top surface 233 links the first and the second side face 231 and 232. It is assumed that the ink covers the first side face 231 and floods its top surface 233. However, it is preferred that the ink stops expanding on the top surface 233 owed to surface tension and as a result does not pour down the second side face 232 which will be kept free from the ink.

Figure 8 shows perspective views of different elevated structures as may be used in a set of elevated structures according to an embodiment of the present invention. Diagram 8a) shows a post with a square base area, diagram 8b) a prism having a triangle base area, diagram 8c) a crown like structure, and diagram 8d) a pillar with a circle base area. In a preferred embodiment, a set of elevated structures as shown in diagram 8c) is provided as outmost elevated structures confining the area, preferably with the tips facing outwards.

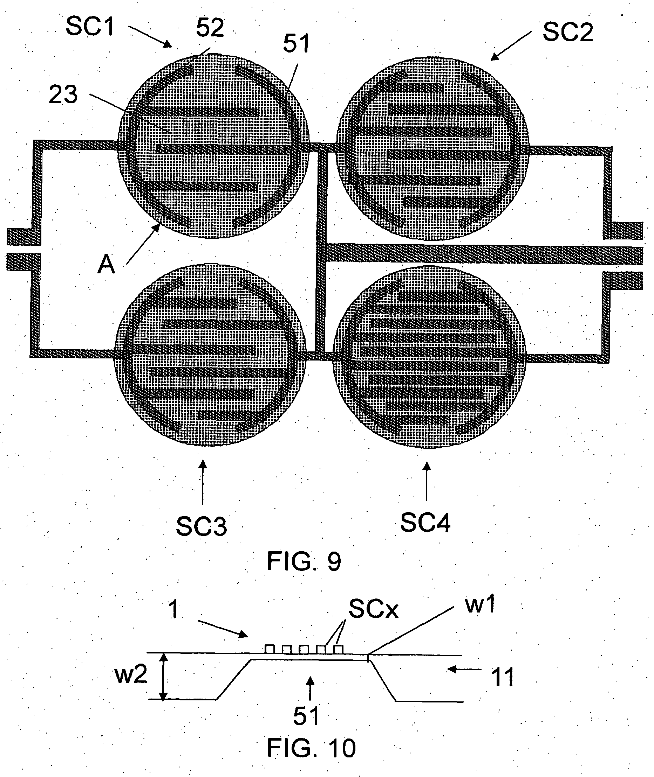

Figure 9 illustrates a top view on a gas sensor according to another embodiment of the present invention. This gas sensor comprises four sensor cells SC1-SC4, each covering a circular maximal admissible coverage area A. The dark structures 51 and 52 denote an electrode pattern for contacting a sensitive film above. The various sensor cells SC1-SC4 may have a different layout of electrode pattern 51, 52.

The area of each sensor cell is covered by multiple individual posts 23. Each small rectangle shalt denote a post 23. The posts 23 are assumed to be arranged on top of the electrode pattern 51, 52, i.e. in an upper level such that in this embodiment the electrodes themselves do not act as expansion inhibitors.

Figure 10 shows a side cut of a gas sensor according to an embodiment of the present invention. Multiple sensor cells SCx are arranged on top of a substrate 1. The substrate 1 has a first thickness w1 in a first region and a second thickness w2 in a second region. The second thickness w2 represents a standard thickness of the substrate 5. In the first region where the sensor cells SCx reside, the substrate 1 is thinned to the first thickness w1. In case that the sensor cells SCx include metal oxide sensitive films, such metal oxide sensitive films may be heated prior to taking a reading. In view of an improved heat efficiency, the sensor cells SCx may be arranged in the first region of the substrate 1 with the smaller thickness w1. The first region with the small thickness w1 may be formed by forming a recess 51 into the backside of the substrate 5.

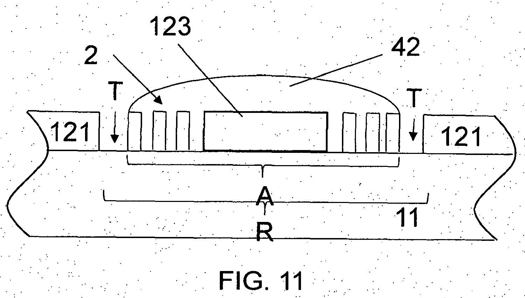

Figure 11 illustrates a sectional view of a gas sensor according to another embodiment of the present invention, which gas sensor is very similar to the gas sensor of Figure 1c) However, another rim 123 is built from the insulating layer within the maximal admissible coverage area A of the substrate. The sensitive material in this example is preferably dispensed onto the rim 123 while in the embodiment of Figure 1c) the sensitive material is preferably dispensed to a different level, i.e. the level of the bulk material.

For all embodiments of the gas sensor, electronic circuitry may preferably be integrated in the subject substrate, specifically by using CMOS processes. Such electronic circuitry may include linearizing, compensating, evaluating, digitizing and/or other functionality.

While there are shown and described presently preferred embodiments of the invention, it is to be distinctly understood that the invention is not limited thereto but may be otherwise variously embodied and practised within the scope of the following claims.

## Claims

1. Gas sensor, comprising
a set of one or more sensor cells (SC), and
a substrate (1),
wherein each sensor cell (SC) of the set comprises:
a sensitive film (42) built from a sensitive material (4) covering an area of the substrate (1),
one or more elevated structures (2) in or around said area for preventing the sensitive material (4) to expand when being applied to said area,
wherein an elevated structure (2) is an elevation from a level of the substrate (1) from which the elevated structure (2) protrudes and to which level the sensitive material (4) reaches down,
wherein one or a number of or all of the elevated structures (2) are made from an insulating coating (12) of the substrate (1).

2. Gas sensor according to claim 1, wherein the one or the number of or all of the elevated structures (2) that are made from an insulating coating (12) are made from one of silicon nitride and silicon oxide.

3. Gas sensor according to claim 1 or claim 2,
wherein the sensitive film (42) emerges from a recess (R) in the insulating coating (12), and
wherein the sensitive film (42) is separated by a trench (T) from a rim (121) of the insulating coating (12) defining the recess (R).

4. Gas sensor according to any one of the preceding claims,
wherein each sensor cell (SC) of the set comprises an electrode pattern for measuring an ohmic resistance of the sensitive film (42),
wherein a number of the one or more elevated structures (2) made from the insulating coating (12) is arranged outside a region covered by the electrode pattern.

5. Gas sensor according to any one of the preceding claims,
wherein one or a number of the elevated structures (2) are made from or covered by an electrically conducting material (13), and in particular are made from or covered by metal, and
in particular wherein one or more of the elevated structures (2) made from or covered by the electrically conducting material represent one or more electrodes for measuring an ohmic resistance of the sensitive film (42).

6. Gas sensor according to claim 4,
wherein each sensor cell (SC) of the set comprises:
at least two elevated structures (2),
wherein at least another one of the elevated structures (2) is made from or covered by the electrically conducting material (13).

7. Gas sensor according to claim 6,
wherein each sensor cell (SC) comprises an inner portion represented by the region covered by the electrode pattern surrounded by an outer portion, and
wherein the at least other one of the elevated structures (2) is represented by the electrode pattern arranged in the inner portion.

8. Gas sensor according to claim 1,
wherein each sensor cell (SC) of the set comprises more than two elevated structures (2).

9. Gas sensor according to claim 8,
wherein each sensor cell (SC) of the set comprises at least three elevated structures (2) in form of a ring (21) each, and
wherein the multiple rings (21) are of different size each and are arranged within each other.

10. Gas sensor according to claim 8 or claim 9,
wherein each sensor cell (SC) of the set comprises a set of more than ten elevated structures (2), and in particular of more than fifty elevated structures (2), and
in particular wherein a footprint of an elevated structure (2) of the set is less than 5 µm x 5 µm, and in particular is less than 1 µm x 1 µm.

11. Gas sensor according to claim 10, wherein each elevated structure (2) of the set has a form of one of: a pillar, a post (23), a prism.

12. Gas sensor according to claim 10 or claim 11,
wherein a footprint of each elevated structure (2) of the set is of one of: an oval, a circle, a rectangle, a square, a triangle.

13. Gas sensor according to any one of the preceding claims,
wherein at least one of the elevated structures (2) comprises a first side face (231) facing the sensitive material (4) when applied to the area (A), a second side face (232) averted from the first side face (231), and a top surface (233) linking the first and the second side face (231, 232),
wherein the first side face (231) and the top surface (233) are covered by the sensitive material (4) while the second side face (232) is not.

14. Method for manufacturing a gas sensor with at least one sensor cell, comprising the steps of
providing a substrate (1) including a maximal admissible coverage area (A) to build the sensor cell (SC) on,
fabricating at least one elevated structure (2) in said maximal admissible coverage area (A) for preventing a sensitive material (4) to expand from said maximal admissible coverage area (A) when being applied thereto, wherein an elevated structure (2) is an elevation from a level of the substrate (1) from which the elevated structure (2) protrudes and to which level the sensitive material (4) reaches down, wherein one or a number of or all of the elevated structures (2) are made from an insulating coating (12) of the substrate (1), and
building a sensitive film (42) of the sensor cell (SC) by contactless dispensing the sensitive material (4) to said maximal admissible coverage area (A), the sensitive material (4) extending and being prevented from expanding from said maximal admissible coverage area (A) by the at least one elevated structure (2).

15. Method according to claim 14,
wherein generating at least one of the one or more elevated structures (2) includes etching an insulating coating (12) of the substrate (1).

16. Method according to claim 14,
wherein generating at least one of the one or more elevated structures (2) includes etching an electrically conductive layer (13) of the substrate (1).

17. Method according to a combination of claims 15 and 16,
wherein between the two etching steps an insulating coating or an electrically conducting material respectively is deposited in the space generated by the first etching step.

## Patentansprüche

1. Gassensor enthaltend
ein Set von einer oder mehreren Sensorzellen (SC) und
ein Substrat (1),
wobei jede Sensorzelle (SC) des Sets enthält:
eine sensitive Schicht (42) aus einem sensitiven Material (4) einen Bereich des Substrats (1) bedeckend,
eine oder mehrere erhöhte Strukturen (2) in oder um besagten Bereich, zum Verhindern der Ausbreitung des sensitiven Materials (4) wenn es auf besagten Bereich aufgetragen wird,
wobei eine erhöhte Struktur (2) eine Erhöhung von einem Niveau des Substrats (1) ist, von welchem die erhöhte Struktur (2) herausragt und zu welchem Niveau das sensitive Material (4) hinunter reicht,
wobei eine oder eine Anzahl oder alle der erhöhten Strukturen (2) aus einer isolierenden Beschichtung (12) des Substrats (1) hergestellt sind.

2. Gassensor nach Anspruch 1,
wobei die eine oder die Anzahl oder alle der erhöhten Strukturen (2), welche aus einer isolierenden Beschichtung (12) hergestellt sind, aus einem von Siliziumnitrid oder Siliziumoxid hergestellt sind.

3. Gassensor nach Anspruch 1 oder Anspruch 2,
wobei die sensitive Schicht (42) aus einer Vertiefung (R) in der isolierenden Beschichtung (12) heraus ragt, und
wobei die sensitive Schicht (42) durch einen Graben (T) von einem Rand (121) der isolierenden Beschichtung (12) separiert ist, welcher die Vertiefung (R) definiert.

4. Gassensor nach einem der vorhergehenden Ansprüche,
wobei jede Sensorzelle (SC) des Sets ein Elektrodenmuster zum Messen eines Ohm'schen Widerstands der sensitiven Schicht (42) hat,
wobei eine Anzahl der einen oder mehreren erhöhten Strukturen (2), welche aus der isolierenden Beschichtung (12) hergestellt sind, ausserhalb eines Bereichs angeordnet sind, welcher durch das Elektrodenmuster bedeckt ist.

5. Gassensor nach einem der vorhergehenden Ansprüche,
wobei eine oder eine Anzahl der erhöhten Strukturen (2) aus einem elektrisch leitenden Material (13) hergestellt oder davon bedeckt sind, und insbesondere aus Metall hergestellt sind oder davon bedeckt sind, und
insbesondere wobei eine oder mehrere der erhöhten Strukturen (2), welche aus dem elektrisch leitenden Material hergestellt sind oder davon bedeckt sind, eine oder mehrere Elektroden verkörpern, zum Messen eines Ohm'schen Widerstands der sensitiven Schicht (42).

6. Gassensor nach Anspruch 4,
wobei jede Sensorzelle (SC) des Sets enthält:
mindestens zwei erhöhte Strukturen (2),
wobei mindestens eine andere der erhöhten Strukturen (2) aus dem elektrisch leitenden Material (13) hergestellt ist oder davon bedeckt ist.

7. Gassensor nach Anspruch 6,
wobei jede Sensorzelle (SC) einen inneren Abschnitt enthält, welcher durch den Bereich verkörpert wird, welcher mit einem Elektrodenmuster bedeckt ist und umgeben ist von einem äusseren Abschnitt, und
wobei die mindestens eine andere der erhöhten Strukturen (2) durch das Elektrodenmuster dargestellt wird, welches im inneren Abschnitt angeordnet ist.

8. Gassensor nach Anspruch 1,
wobei jede Sensorzelle (SC) des Sets mehr als zwei erhöhte Strukturen (2) enthält.

9. Gassensor nach Anspruch 8,
wobei jede Sensorzelle (SC) des Sets mindestens drei erhöhte Strukturen (2), jede in Form eines Rings, enthält, und
wobei die mehreren Ringe (21) unterschiedlich gross sind und ineinander angeordnet sind.

10. Gassensor nach Anspruch 8 oder Anspruch 9,
wobei jede Sensorzelle (SC) des Sets ein Set von mehr als zehn erhöhten Strukturen (2) enthält und insbesondere von mehr als fünfzig erhöhten Strukturen (2) und
insbesondere, wobei eine Grundfläche einer erhöhten Struktur (2) des Sets weniger als 5µm x 5µm ist, und insbesondere weniger als 1µm x 1µm.

11. Gassensor nach Anspruch 10,
wobei jede erhöhte Struktur (2) des Sets die Form hat von einem aus: eine Säule, ein Pfosten (23), ein Prisma.

12. Gassensor nach Anspruch 10 oder Anspruch 11, wobei eine Grundfläche jeder erhöhten Struktur (2) des Sets eine ist von: ein Oval, ein Kreis, ein Rechteck, ein Quadrat, ein Dreieck.

13. Gassensor nach einem der vorhergehenden Ansprüche,
wobei mindestens eine der erhöhten Strukturen (2) eine erste Seitenfläche (231) enthält, welche dem sensitiven Material (4) zugewandt ist wenn in dem Bereich (A) aufgetragen, eine zweite Seitenfläche (232), welche von der ersten Seitenfläche (231) abgewandt ist und eine obere Oberfläche (233), welche die erste und die zweite Seitenfläche (231, 232) verbindet,
wobei die erste Seitenfläche (231) und die obere Oberfläche (233) mit dem sensitiven Material (4) bedeckt sind während die zweite Seitenfläche (232) das nicht ist.

14. Verfahren zur Herstellung eines Gassensors mit mindestens einer Sensorzelle, enthaltend die Schritte
Bereitstellen eines Substrats (1) beinhaltend einen maximal zulässigen Abdeckbereich (A) zum Bilden der Sensorzelle (SC) darauf,
Fabrizieren mindestens einer erhöhten Struktur (2) in besagtem maximal zulässigen Abdeckbereich (A) zum Verhindern der Ausbreitung eines sensitiven Materials (4) über den maximal zulässigen Abdeckbereich (A), wenn es darauf appliziert wird, wobei eine erhöhte Struktur (2) eine Erhöhung ist von einem Niveau des Substrats (1), von welchem die erhöhte Struktur (2) hervorsteht und zu welchem Niveau das sensitive Material (4) hinunter reicht, wobei eine oder eine Anzahl oder alle der erhöhten Strukturen (2) aus einer isolierenden Beschichtung (12) des Substrats (1) hergestellt sind, und
Bilden einer sensitiven Schicht (42) der Sensorzelle (SC) mittels kontaktlosem Dispensieren des sensitiven Materials (4) zu besagtem maximal zulässigen Abdeckbereich (A), wobei sich das sensitive Material (4) ausbreitet und wobei die mindestens eine erhöhte Struktur (2) verhindert, dass sich das Material (3) über den maximal zulässigen Abdeckbereich (A) hinaus ausbreitet.

15. Verfahren nach Anspruch 14,
wobei das Generieren mindestens einer der einen oder mehreren erhöhten Strukturen (2) das Ätzen einer isolierenden Beschichtung (12) des Substrats (1) enthält.

16. Verfahren nach Anspruch 14,
wobei das Generieren mindestens einer der einen oder mehreren erhöhten Strukturen (2) das Ätzen einer elektrisch leitfähigen Schicht (13) des Substrats (1) enthält.

17. Verfahren nach einer Kombination der Ansprüche 15 und 16,
wobei zwischen den zwei Ätzschritten eine isolierende Beschichtung respektive ein elektrisch leitfähiges Material in den Raum deponiert wird, welcher durch den ersten Ätzschritt generiert wird.

## Revendications

1. Capteur de gaz, comprenant
un set d'une ou plusieurs cellules de capteur (SC), et
un substrat (1),
chaque cellule de capteur (SC) du set comprenant:
une pellicule sensible (42) formée d'un matériau sensible (4) qui couvre une zone du substrat (1),
une ou plusieurs structures (2) à l'intérieur ou autour de ladite zone pour prévenir que le matériau sensible (4) s'élargit quand il est appliqué sur ladite zone,
une structure élevée (2) étant une élévation d'un niveau du substrat (1) à partir duquel la structure élevée (2) fait saille et vers lequel le matériau sensible (4) s'étend vers le bas,
une ou plusieurs ou toutes les structures élevées (2) étant fabriquées d'un revêtement d'isolation (12) du substrat (1).

2. Capteur de gaz selon la revendication 1, la structure ou les plusieurs structures ou toutes les structures élevées (2) fabriquées d'un revêtement d'isolation (12) étant fabriquées d'un de: nitrure de silicium et oxyde de silicium.

3. Capteur de gaz selon la revendication 1 ou la revendication 2,
la pellicule sensible (42) sortant d'une cavité (R) dans le revêtement d'isolation (12), et
la pellicule sensible (42) étant séparée par une fente (T) d'une bordure (121) du revêtement d'isolation (12) qui définit la cavité (R).

4. Capteur de gaz selon l'une des revendications précédentes,
chaque cellule de capteur (SC) du set comprenant un modèle d'électrode pour mesurer une résistance ohmique de la pellicule sensible (42),
un nombre de l'une ou des plusieurs structures élevées (2) fabriquées du revêtement d'isolation (12) étant arrangé hors de la zone couverte par le modèle d'électrode.

5. Capteur de gaz selon l'une des revendications précédentes,
une ou un nombre des structures élevées (2) étant fabriquées d'un ou couvertes par un matériau électriquement conducteur (13), et particulièrement étant fabriquées d'un ou couvertes par un métal, et
particulièrement une ou un nombre des structures élevées (2) fabriquées du ou couvertes par le matériau électriquement conducteur (13) représentant une ou plusieurs électrodes pour mesurer une résistance ohmique de la pellicule sensible (42).

6. Capteur de gaz selon la revendication 4, chaque cellule de capteur (SC) du set comprenant:
au moins deux structures élevées (2),
au moins une autre des structures élevées (2) étant fabriquée du ou couverte par le matériau électriquement conducteur (13).

7. Capteur de gaz selon la revendication 6, chaque cellule de capteur (SC) comprenant une portion intérieure représentée par la zone couverte par le modèle d'électrode entouré par une portion extérieure, et
au moins l'autre des structures élevées (2) étant représentée par un modèle d'électrode arrangé dans la portion intérieure.

8. Capteur de gaz selon la revendication 1, chaque cellule de capteur (SC) du set comprenant plus que deux structures élevées (2).

9. Capteur de gaz selon la revendication 8,
chaque cellule de capteur (SC) du set comprenant au moins trois structures élevées (2) chaque en forme d'un anneau (21), et
les plusieurs anneaux (21) ayant des largeurs différentes et étant arrangées l'une dans l'autre.

10. Capteur de gaz selon la revendication 8 ou la revendication 9,
chaque cellule de capteur (SC) du set comprenant un set de plus de dix structures élevées (2), et particulièrement de plus de cinquante structures élevées (2), et
particulièrement une empreinte d'une structure élevée (2) du set étant inférieure à 5 µm x 5 µm, et particulièrement inférieure à 1 µm x 1 µm.

11. Capteur de gaz selon la revendication 10, chaque structure élevée (2) du set ayant une forme d'un de: un pilier, une barre (23), une prisme.

12. Capteur de gaz selon la revendication 10 ou la revendication 11,
une empreinte de chaque structure élevée (2) du set étant une de: ovale, cercle, rectangle, carré, triangle.

13. Capteur de gaz selon l'une des revendications précédentes,
au moins une des structures élevées (2) comprenant une face d'un premier côté (231) qui est en face du matériau sensible (4) quand appliqué dans la zone (A), une face d'un deuxième côté (232) éloignée de la face du premier côté (231), et une surface supérieure (233) qui relie la face du premier et du deuxième côté (231, 232),
la face du premier côté (231) et la face supérieure (233) étant couvertes par le matériau sensible (4) alors que la face du deuxième côté n'y est pas.

14. Procédé pour fabriquer un capteur de gaz avec au moins une cellule de capteur, comprenant les étapes:
de prévoir un substrat (1) incluant une zone de couverture (A) admissible maximale afin de former sur elle la cellule de capteur (SC),
de fabriquer au moins une structure élevée (2) dans la zone de couverture (A) admissible maximale afin d'empêcher un matériau sensible (4) de s'étendre au-delà de ladite zone de couverture (A) admissible maximale quand il y est appliqué, une structure élevée (2) étant une élévation d'un niveau du substrat (1) duquel la structure élevée (2) fait saille et vers lequel le matériau sensible (4) s'étend vers le bas, une ou plusieurs ou toutes les structures élevées (2) étant fabriquées d'un revêtement d'isolation (12) du substrat (1), et
de former une pellicule sensible (42) de la cellule de capteur (SC) en distribuant le matériau sensible (4) sans-contact à ladite zone de couverture (A) admissible maximale, le matériau sensible (4) s'étendant et étant empêché de s'étendre au-delà de ladite zone de couverture (A) admissible maximale par au moins l'une structure élevée (2).

15. Procédé selon la revendication 14,
la génération d'au moins une de l'une ou des plusieurs structures élevées (2) incluant la gravure d'un revêtement d'isolation (12) sur le substrat (1).

16. Procédé selon la revendication 14,
l'étape de génération d'au moins une de l'une ou des plusieurs structures élevées (2) incluant la gravure d'une couche électriquement conductrice (13) du substrat (1).

17. Procédé selon une combinaison des revendications 15 et 16,
entre les deux étapes de gravure un revêtement d'isolation ou bien un matériau électriquement conducteur étant déposé dans l'espace généré par la première étape de gravure.
